(19) Europäisches Patentamt
European Patent Office
Office européen des brevets

(11) Numéro de publication: **0 062 587**
**A1**

(12) # DEMANDE DE BREVET EUROPEEN

(21) Numéro de dépôt: **82400619.1**

(22) Date de dépôt: **05.04.82**

(51) Int. Cl.³: **C 07 D 233/52**, C 07 D 239/18, C 07 D 401/12, C 07 D 403/12, C 07 D 409/12, A 01 N 43/50, A 01 N 43/54

(30) Priorité: **16.03.82 FR 8204436**
**06.04.81 FR 8106866**

(43) Date de publication de la demande: **13.10.82**
**Bulletin 82/41**

(84) Etats contractants désignés: **AT BE CH DE FR GB IT LI LU NL SE**

(71) Demandeur: **HEXACHIMIE Société anonyme dite:, 128, rue Danton, F-92500 Rueil-Malmaison (FR)**

(72) Inventeur: **Cognacq, Jean-Claude, 22, rue du Buat, F-78580 Maule (FR)**
Inventeur: **Teulon, Jean-Marie, 12, Résidence du Bel Ebat 56, avenue de Verdun, F-78170 La Celle Saint Cloud (FR)**
Inventeur: **Bouley, Etienne, 19, rue Barbes, F-92400 Courbevoie (FR)**
Inventeur: **Lacrampe, Jean, 4, rue Edith Cavell, F-92400 Courbevoie (FR)**
Inventeur: **Rambeaux, Jacques, 54, rue de Picpus, F-75012 Paris (FR)**

(74) Mandataire: **Richebourg, Michel François, Cabinet Beau de Loménie 55, rue d'Amsterdam, F-75008 Paris (FR)**

(54) **Nouveaux dérivés de l'hydrazine, leur préparation et leur utilisation comme fongicides et bactéricides.**

(57) L'invention concerne de nouveaux composés de formule:

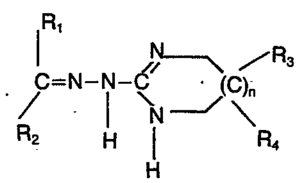

(I)

Fongicides à 50–2000 g/ha environ, et bactéricides. Action, entre autres, sur la rouille du haricot.

EP 0 062 587 A1

Nouveaux dérivés de l'hydrazine, leur préparation et leur utilisation comme fongicides et bactéricides.

La présente invention se rapporte à de nouveaux dérivés de l'hydrazine, leur procédé de préparation et leur application notamment comme fongicides et bactéricides.

Ces nouveaux composés répondent à la formule générale (I)

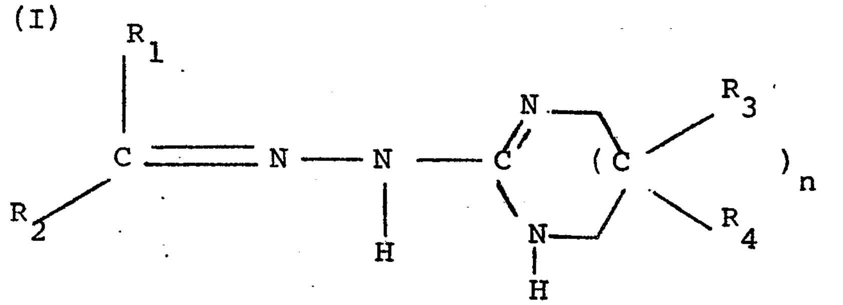

(I)

dans laquelle :

$R_1$ représente

- un radical alkyle linéaire ou ramifié de 1 à 18 atomes de carbone ou un radical cyclo alkyle comprenant de 3 à 6 atomes de carbone, pouvant être substitué par un radical alkyle inférieur ;
- un groupement aryle de formule

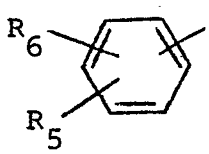

dans laquelle $R_5$ et $R_6$, qui sont identiques ou différents, représentent un atome d'hydrogène, un radical alkyle linéaire ou ramifié de 1 à20 atomes de carbone, un radical alkyloxy, un radical alkylthio, un radical alkényloxy, un radical cycloalkyle, un radical trifluorométhyle, un atome d'halogène, un radical aryle substitué ou non, un groupement aryloxy substitué ou non ;
- un groupement indanyl-5 substitué ou non en position 2 par un radical alkyle inférieur linéaire ou ramifié ;
- un radical naphtyl-1 ou naphtyl-2 substitué ou non par

un groupement alkyle ou alkyloxy inférieur ;
- ou bien un hétérocycle azoté comme la pyridine ou la pyrimidine, substitué ou non par les radicaux $R_5$ et $R_6$ définis comme ci-dessus ;
- ou bien un hétérocycle soufré comme le cyclopentathiophène substitué ou non par un radical alkyle inférieur ;
$R_2$ représente :
- un atome d'hydrogène ou un radical alkyle inférieur ;
$R_1$ et $R_2$ pouvant former ensemble avec l'atome de carbone auquel ils sont reliés un cycloalkane, avantageusement le cyclopentane ou le cyclohexane, substitué ou non par un radical alkyle linéaire ou ramifié de 1 à 5 atomes de carbone ;
n vaut zéro ou un,
et, dans le cas où n = 1 :
$R_3$ et $R_4$, identiques ou différents, représentent un atome d'hydrogène ou un radical alkyle inférieur.

Les produits préférés selon l'invention sont ceux pour lesquels :
$R_1$ représente :
- un groupe aryle dans lequel $R_5$ représente un radical alkyle inférieur ou un radical cycloalkyle et $R_6$ représente un atome d'hydrogène ;
- un groupement indanyl-5 substitué en 2 par un radical alkyle inférieur ;
- un radical naphtyl-2 substitué en 6 par un groupement méthoxy ;
$R_2$ représente :
- un groupement méthyle;
n est : égal à zéro.

Lorsque $R_1$ représente un groupe aryle, des composés tout à fait préférés sont ceux qui présentent

une chaîne isobutyle ou tert-butyle en position para, $R_2$ réprésentant un radical alkyle.

Une classe de composés particulièrement préférés est celle dans laquelle $R_1$ représente un groupe indanyl-5 éventuellement substitué.

Une sous-famille intéressante concerne des composés de formule :

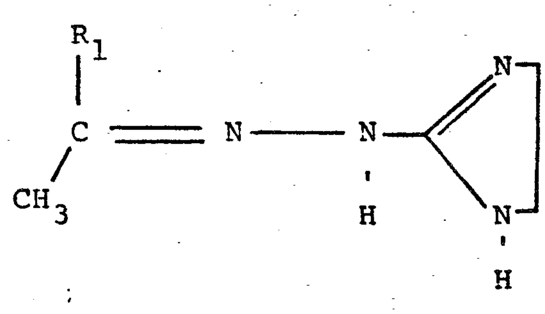

(I')

dans laquelle

$R_1$ est : un groupement aryle de formule :

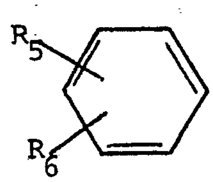

dans laquelle $R_5$ et $R_6$ qui sont identiques ou différents, représentent un atome d'hydrogène ou un radical alkyle linéaire ou ramifié de 5 à 20 atomes de carbone, ou un radical cycloalkyle.

Les produits préférés de cette sous famille sont ceux dans lesquels :

$R_1$ est : un groupe aryle dans lequel $R_5$ représente un radical alkyle linéaire ou ramifié de 5 à 20 atomes de carbone ou un radical cycloalkyle, et $R_6$ l'atome d'hydrogène.

De préférence $R_5$ sera en position para.

De manière encore préférée, $R_5$ est en para et $R_6 = H$.

Les produits concernés sont systémiques, c'est-à-dire qu'ils sont véhiculés par la sève.

L'invention couvre également les sels d'acides minéraux et organiques de ces dérivés, tels que chlorhydrate, bromhydrate, phosphate, acétate, maléate, etc.

On peut préparer, selon l'invention, les composés de formule (I) ou (I') par action d'un composé de formule (II) sur un composé de formule (III) dans un solvant organique, choisi principalement parmi les alcools et l'éthylèneglycol, à des températures comprises entre 60 et 130° C :

(II) 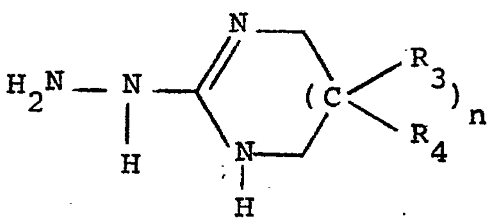

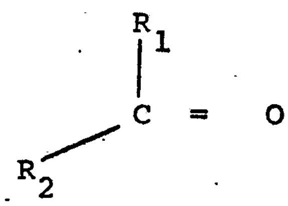 (III)

($R_1$, $R_2$, $R_3$, $R_4$ et n sont définis comme ci-dessus).

Les composés de formule générale (I), et leurs sels, ont des propriétés fongicides ou fongistatiques et bactéricides et, à ce titre, peuvent être employés, pour la protection des plantes cultivées, contre les attaques de certains champignons parasites, qu'ils détruisent ou dont ils limitent la progression. Comme champignons parasites sur lesquels les composés selon l'invention sont actifs, on peut citer en particulier Fusarium roseum, Rhizoctonia solani, Phomopsis viticola, Alternaria tenuis, Uromyces phaseoli,Peronospora viticola, Botrytis fabae, Botrytis cinerea, Penicillium expansum, Aspergillus niger, Phytophthora infestans, Erysiphe graminis, Puccinia striiformis (rouille du blé), Cercosporoella herpotrichoïtes (piétin)...

Pour leur mise en oeuvre, les composés fongicides selon l'invention sont incorporés dans des formulations qui contiennent, outre la matière active, les

additifs inertes habituellement utilisés en agriculture pour faciliter la conservation, la mise en suspension aqueuse, l'adhérence sur les plantes et la résistance aux agents atmosphériques et aux dégradations, tels que diluants solides (talc, argile, craie, silice, etc.) ou liquides (eau, huiles minérales, solvants organiques), agents tensio-actifs, etc. De telles formulations peuvent se présenter sous la forme de poudres mouillables, poudres pour poudrage, suspensions aqueuses, solutions dans des solvants organiques, etc. Elles peuvent contenir de 1 % à 99 % en poids, de préférence de 5 % à 90 % en poids, de matière active.

Les doses de composés selon l'invention appliquées lors du traitement fongicide des plantes cultivées sont en général comprises entre 50 et 2000 g par hectare.

Les exemples suivants illustrent l'invention sans toutefois en limiter la portée.

Le Tableau IX ci-après rassemble les formules des composés apparaissant dans ces exemples.

EXEMPLE 1 :

N- /‾(isopropyl-2' indanyl-5')-1 méthylène_7
N'- (imidazolino-2) hydrazine

Formule (I) : $R_1$ = isopropyl-2 indanyl-5,
$R_2$ = H
n = O

14,7 g d'isopropyl-2 formyl-5 indane (0,078 mole) dans 100 ml d'éthanol sont portés au reflux pendant 5 heures en présence de 14,1 g (0,078 mole) de bromhydrate d'hydrazino-2 imidazoline-2.

La solution est refroidie à température ambiante et agitée à cette température pendant 1 heure. Le bromhydrate du produit attendu précipite ; il est essoré, lavé à l'éthanol, puis à l'éther. On obtient 17,6 g du bromhydrate de N - [(isopropyl-2' indanyl-5')-1 méthylène] N'-(imidazolino-2) hydrazine brut fondant à 193° C ($R^{dt}$ = 65 % brut).

Le bromhydrate mis en suspension dans l'eau, est traité par une solution d'ammoniaque pendant 1 heure.

Le produit est essoré, rincé abondamment à l'eau, et séché. On obtient 13 g de N - [(isopropyl-2' indanyl-5')-1 méthylène] N'-(imidazolino-2) hydrazine fondant à 218° C ($R^{dt}$ = 48 %) - Pureté (dosage perchlorique) : 100 %.

<u>EXEMPLE 2</u> :

N-[(isopropyl-2' indanyl-5')-1 éthylidène] N'-(imidazolino-2) hydrazine.

<u>Formule (I)</u> : $R_1$ = isopropyl-2 indanyl-5
$R_2$ = méthyl
n = 0

Même mode opératoire que dans l'Exemple 1 mais à partir de 15,1 g d'isopropyl-2 acétyl-5 indane (0,075 mole), 13,6 g de bromhydrate d'hydrazino-2 imidazoline-2 (0,075 mole) dans 100 ml d'éthylène glycol, à 120° C pendant 6 heures.

Après un traitement à l'ammoniaque identique à celui décrit à l'Exemple 1 on obtient 14 g de N - [(isopropyl-2' indanyl-5')-1 éthylidène] N'-(imidazolino-2) hydrazine, fondant à 210° C ($R^{dt}$ = 52 %)-Pureté (dosage perchlorique) : 98,3 %.

EXEMPLE 3 :

N - /‾(isopropyl-2' indanyl-5')-1 propylidène/ N'- (imidazolino-2) hydrazine, et maléate correspondant.

Formule (I) : $R_1$ = isopropyl-2 indanyl-5
$R_2$ = éthyl
n = 0

a) 33 g d'isopropyl-2 propionyl-5 indane (0,15 mole), 27 g de bromhydrate d'hydrazino-2 imidazoline-2 (0,15 mole) dans 100 ml d'éthylène glycol sont portés à 120° C pendant 6 heures.

La solution obtenue est refroidie, diluée à l'eau, le produit obtenu est essoré, lavé à l'éthanol et séché.

On obtient 31 g de bromhydrate de N - /‾(isopropyl-2' indanyl-5')-1 propylidène/ N' - (imidazolino-2) hydrazine brut fondant à 214° C ($R^{dt}$ = 55 %).

b) Préparation du maléate :

Les eaux-mères du bromhydrate sont alcalinisées à l'ammoniaque, extraites au chlorure de méthylène. La phase organique est lavée à l'eau, séchée puis filtrée et le solvant est évaporé.

On obtient 19,8 g de base brute sous forme d'huile que l'on met en solution dans 100 ml d'éthanol en présence de 8 g d'acide maléique. On obtient ainsi 10,8 g de maléate de N - /‾(isopropyl-2' indanyl-5')- 1 propylidène/ N' - (imidazolino-2) hydrazine fondant à 204° C - Pureté (dosage perchlorique) 99,3 %.

c) Préparation de la base :

Les 31 g de bromhydrate précédemment obtenus

sont mis en suspension dans l'eau et traités à l'ammoniaque pendant 1 heure.

Après essorage du précipité, lavage à l'eau et séchage, on obtient 23,2 g de N - /(isopropyl-2' indanyl-5')-1 propylidène/ N' - (imidazolino-2) hydrazine fondant à 160 ° C - Pureté (dosage perchlorique) : 99,8 %.

EXEMPLE 4 :

Acétate de N - /(indanyl-5')-1 éthylidène/ N'-(imidazolino-2) hydrazine.

Formule (I) : $R_1$ = indanyl-5
$R_2$ = méthyl
n = O

a) Même mode opératoire que celui décrit dans l'Exemple 1 mais à partir de 16,3 g d'acétyl-5 indane (0,101 mole), 18 g de bromhydrate d'hydrazino-2 imidazoline-2 (0,1 mole), et 50 ml d'éthylène glycol, à 120° C pendant 6 heures.

On obtient 22,3 g de bromhydrate de N-/(indanyl-5')-1 éthylidène/ N' - (imidazolino-2) hydrazine brut ($R^{dt}$ = 68 % brut). Le traitement à l'ammoniaque selon l'Exemple 1 permet d'obtenir 15,4 g de base brute libérée de son sel, et fondant à 170° C. ($R^{dt}$ = 92 % à partir du bromhydrate).

b) Préparation de l'acétate :

Les 15,4 g de base brute précédemment obtenus sont mis en solution dans 50 ml de méthanol contenant 3,5 ml d'acide acétique. Après évaporation du solvant, le résidu est repris par 50 ml d'acétone. Le précipité est essoré, lavé à l'acétone et à l'éther puis séché.

On obtient 14,6 g de composé brut ($R^{dt}$ = 76 % brut) que l'on recristallise dans l'éthanol, pour donner 10 g d'acétate de N - /(indanyl-5')-1 éthylidène/ N' - (imidazolino-2) hydrazine fondant à 190° C - Pureté (dosage perchlorique) 98 %, $R^{dt}$ de la recristallisation = 68 % - $R^{dt}$ global = 33 %.

EXEMPLE 5 :

Acétate de N- /(indanyl-5')-1 propylidène/ N' - (imidazolino-2) hydrazine.

Formule (I) : $R_1$ = indanyl-5
$R_2$ = éthyl
n = 0

a) Même mode opératoire que celui décrit dans l'exemple 1 mais à partir de 9,4 g de propionyl-5 indane (0,054 mole), 9,1 g de bromhydrate d'hydrazino-2 imidazoline-2 (0,05 mole) dans 50 ml d'éthylène glycol à 120° C pendant 6 heures.

Le traitement à l'ammoniaque selon l'Exemple 1 permet d'obtenir 6,6 g de N-/(indanyl-5')-1 propylidène/ N' - (imidazolino-2) hydrazine que l'on recristallise dans l'éther à -30° C et qui fond à 180° C. ($R^{dt}$ = 48 %).

b) Préparation de l'acétate :

Même mode opératoire que celui décrit dans l'Exemple 4 mais à partir de 6,6 g de base précédemment préparée, 20 ml de méthanol et 1,5 ml d'acide acétique.

On obtient 5 g d'acétate de N-/(indanyl-5')-1 propylidène/ N'-(imidazolino-2) hydrazine fondant à 168° C - Pureté (dosage perchlorique) 99,5 %. $R^{dt}$ à partir de la base = 64 % ; $R^{dt}$ global = 31 %.

EXEMPLE 6 :

N-/(terbutyl-4' phényl)-1 éthylidène/ N'-(imidazolino-2) hydrazine, et bromhydrate correspondant.

Formule (I) : $R_1$ = terbutyl-4 phényl
$R_2$ = méthyl
n = O

a) Mode opératoire identique à celui décrit dans l'Exemple 1 mais à partir de 17,7 g de terbutyl-4' phényl méthyl cétone (O,1 mole), 18,1 g de bromhydrate d'hydrazino-2 imidazoline-2 (O,1 mole), dans 125 ml de butanol, pendant 5 heures au reflux.

On isole ainsi 28,7 g de bromhydrate de N-/‾(terbutyl-4' phényl)-1 éthylidène_/ N' - (imidazolino-2) hydrazine fondant à 221° C. ($R^{dt}$ = 85 %) - Pureté (dosage perchlorique) : 98,9 %.

b) Préparation de la base :

Le traitement à l'ammoniaque selon l'Exemple 1 sur 14,7 g du bromhydrate précédemment isolé, donne 8 g de N - /‾(terbutyl-4' phényl) - 1 éthylidène_/ N' - (imidazolino-2) hydrazine fondant à 227° C - Pureté (dosage perchlorique) : 98,8 %. $R^{dt}$ à partir du bromhydrate: 66 %.

EXEMPLE 7 :

N - /‾(isobutyl-4' phényl) - 1 éthylidène_/ N' - (imidazolino-2) hydrazine, et bromhydrate correspondant.

Formule (I) : $R_1$ = isobutyl-4 phényl
$R_2$ = méthyl
n = O

a) Mode opératoire identique à celui décrit dans l'Exemple 1, mais à partir de 13,7 g d'isobutyl-4' phényl méthyl cétone (O,078 mole), 13,6 g de bromhydrate d'hydrazino-2 imidazoline-2 (O,075 mole) dans 100 ml de butanol au reflux pendant 6 heures.

On isole ainsi 19 g de bromhydrate de N - /¯- (isobutyl-4' phényl)-1 éthylidène/ N' - (imidazolino-2) hydrazine, fondant à 270° C ($R^{dt}$ = 72 %)- Pureté (dosage perchlorique) 100,5 %.

b) Préparation de la base :

Le traitement à l'ammoniaque selon l'Exemple 1, sur 10 g du bromhydrate précédent, fournit 7 g de N - /¯(isobutyl-4' phényl)-1 éthylidène/ N' - (imidazolino-2) hydrazine fondant à 172° C - pureté (dosage perchlorique) : 99,5 %. $R^{dt}$ à partir du bromhydrate : 93 %.

EXEMPLE 8 :

N - /¯(cyclohexyl-4' phényl)-1 éthylidène/
N'-(imidazolino-2) hydrazine, et bromhydrate
correspondant.

Formule (I) : $R_1$ = cyclohexyl-4 phényl
$R_2$ = méthyl
n = O

a) Mode opératoire identique à celui de l'Exemple 1 mais à partir de 5,6 g de cyclohexyl-4' phényl méthyl cétone (0,028 mole), 4,9 g de bromhydrate d'hydrazino-2 imidazoline-2 (0,027 mole) dans 75 ml de butanol au reflux pendant 5 heures.

On isole ainsi 7,8 g de bromhydrate de N-/¯(cyclohexyl-4' phényl)-1 éthylidène/ N' - (imidazolino-2) hydrazine fondant à une température supérieure à 250° C - Pureté (dosage perchlorique) : 100,1 %. $R^{dt}$ = 76 %. F (appareil Totoli) 264-5° C.

b) Préparation de la base :

A partir de 5,8 g du bromhydrate précédemment isolé, et par le traitement à l'ammoniaque selon l'Exemple 1,

on obtient 4,5 g de N -/¯(cyclohexyl-4' phényl)-1 éthylidène/ N'-(imidazolino-2) hydrazine fondant à 214 ° C Pureté (dosage perchlorique) : 99,8 %. $R^{dt}$ à partir du bromhydrate = 100 %.

EXEMPLE 9 :

N - /¯(diphénylyl-4')-1 éthylidène/ N' (imidazolino-2) hydrazine, et chlorhydrate correspondant.

Formule (I) : $R_1$ = diphényl-4'

$R_2$ = méthyl

n = 0

a) Mode opératoire identique à celui décrit dans l'Exemple 1 mais à partir de 13,5 g de phényl-4' phényl méthyl cétone (0,0688 mole) 12 g de bromhydrate d'hydrazino-2 imidazoline-2, 50 ml d'éthylène glycol, 120° C pendant 6 heures.

Le traitement à l'ammoniaque selon l'Exemple 1 permet d'isoler 16,8 g de base brute ($R^{dt}$ 87,9 %) fondant à 254° C.

Par recristallisation de 12 g de cette base brute dans 100 ml de DMF on obtient 7,1 g de N- /¯(diphénylyl-4')-1 éthylidène/N' - (imidazolino-2) hydrazine fondant à 255° C - Pureté (dosage perchlorique) : 98,5 %. $R^{dt}$ de la recristallisation : 60 %.

b) Préparation du chlorhydrate :

A une solution de 4,8 g de base brute dans 20 ml de méthanol on ajoute de l'éther chlorhydrique jusqu'à acidité. Le précipité obtenu est filtré et séché.

On obtient 4,5 g de N-/¯(diphénylyl-4')-1 éthylidène/ N'-(imidazolino-2) hydrazine fondant à une température supérieure à 250° C - Pureté (dosage perchlorique) : 99,5 %. $R^{dt}$ = 82 %.

F (appareil Totoli) : 295-6° C.

EXEMPLE 10 :

N - /(phénoxy-4' phényl)-1 éthylidène/ N' - (imidazolino-2) hydrazine, et chlorhydrate correspondant.

Formule (I) : $R_1$ = phénoxy-4' phényl
$R_2$ = méthyl
n = 0

a) Mode opératoire identique à celui décrit dans l'Exemple 1 mais à partir de 21 g (0,099 mole) de phénoxy-4' phényl méthyl cétone, 17,7 g de bromhydrate d'hydrazino-2 imidazoline-2 (0,098 mole), 75 ml d'éthylène glycol, à 120° C pendant 5 heures.

Le traitement à l'ammoniaque permet d'isoler 25,4 g de base brute ($R^{dt}$ = 87 %) que l'on peut recristalliser dans le méthanol pour obtenir 17,2 g de N - /(phénoxy-4' phényl)-1 éthylidène/ N' - (imidazolino-2) hydrazine fondant à 200° C - Pureté (dosage perchlorique) : 99,5 %. $R^{dt}$ de la recristallisation : 68 %.

b) Préparation du chlorhydrate :

7 g de base, en solution dans le méthanol, sont acidifiés par de l'éther chlorhydrique. La solution est évaporée. Le résidu est repris à l'acétone.

Le précipité est filtré et séché.

On obtient 5,2 g de chlorhydrate de N - /(phénoxy-4' phényl)-1 éthylidène/ N' - (imidazolino-2) hydrazine fondant à une température supérieure à 250° C - Pureté (dosage perchlorique) : 99,6 %. $R^{dt}$ = 67 %.
F (appareil Totoli) : 280-2° C.

EXEMPLE 11 :

N-/(naphtyl-1')-1 éthylidène/ N'-(imidazolino-2) hydrazine.

Formule (I) : $R_1$ = napthyl-1
$R_2$ = méthyl
n = O

Mode opératoire identique à celui décrit dans l'Exemple 1 mais à partir de 8,5 g d'acétyl-1 naphtalène (0,05 mole), 9 g de bromhydrate d'hydrazino-2 imidazoline-2 (0,05 mole), dissous dans 20 ml $H_2O$ et 90 ml d'éthanol, au reflux pendant 7 heures.

Après traitement à l'ammoniaque selon l'Exemple I, on isole 10 g de N - /‾(naphtyl-1')-1 éthylidène_7 N' - (imidazolino-2) hydrazine fondant à 165° C ($R^{dt}$ = 79 %) - Pureté (dosage perchlorique) : 97,5 %.

EXEMPLE 12 :

Bromhydrate de N- /‾(méthoxy-6' naphtyl-2')-1 éthylidène_7 N' -(imidazolino-2) hydrazine.

Formule (I) : $R_1$ = méthoxy-6 naphtyl-2
$R_2$ = méthyl
n = O

Mode opératoire identique à celui décrit dans l'Exemple 1 mais à partir de 10 g d'acétyl-2 méthoxy-6 naphtalène (0,05 mole), 9 g de bromhydrate d'hydrazino-2 imidazoline-2 dissous dans 20 ml d'eau, et 100 ml d'éthanol, au reflux pendant 7 heures.

On isole ainsi après traitement à l'ammoniaque selon l'Exemple 1, 5,5 g du bromhydrate de N - /‾(méthoxy-6' naphtyl-2')-1 éthylidène_7 N' - (imidazolino-2) hydrazine fondant à une température supérieure à 260° C - Pureté (dosage perchlorique) : 100,9 %.
F (appareil Totoli) : 292-3° C.

EXEMPLE 13 :

Bromhydrate de N - (isopropylidène) N' - (imidazolino-2) hydrazine.

Formule (I) : $R_1$ = méthyl
$R_2$ = méthyl
n = O

Mode opératoire identique à celui décrit dans l'Exemple 1 mais à partir de 5,8 g d'acétone (O,1 mole), 9,1 g de bromhydrate d'hydrazino-2 imidazoline-2 (O,05 mole) dans 100 ml d'éthanol et 35 ml d'eau, à 100° C pendant 8 heures en autoclave.

On isole ainsi 8,8 g de bromhydrate de N- (isopropylidène) N' - (imidazolino-2) hydrazine fondant à 169° C - Pureté (dosage perchlorique) : 100,6 %. $R^{dt}$ = 79 %.

EXEMPLE 14 :

Bromhydrate de N - /(isobutyl)-1 éthylidène/ N' - (imidazolino-2) hydrazine.

Formule (I) : $R_1$ = isobutyl
$R_2$ = méthyl
n = O

Mode opératoire identique à celui décrit dans l'Exemple 1, mais à partir de 10 g de méthyl isobutylcétone (O,1 mole), 9,1 g de bromhydrate d'hydrazino-2 imidazoline-2, (O,05 mole) 150 ml d'éthanol et 50 ml d'eau, pendant 8 heures à 100° C en autoclave.

Le traitement selon l'Exemple 1 permet d'isoler 10 g de bromhydrate de N - /(isobutyl)-1 éthylidène/ N' - (imidazolino-2) hydrazine fondant à 72° C - Pureté (dosage perchlorique) : 98,2 %. $R^{dt}$ = 76 %.

EXEMPLE 15 :

Bromhydrate de N- /(n-pentyl)-1 éthylidène/ N'- (imidazolino-2) hydrazine

Formule (I) : $R_1$ = n-pentyl
$R_2$ = méthyl
n = O

Mode opératoire identique à celui décrit dans l'Exemple 1, mais à partir de 14 ml de méthyl amyl cétone, 9,1 g de bromhydrate d'hydrazino-2 imidazoline-2, 100 ml d'éthanol et 35 ml d'eau, pendant 8 heures à 100° C en autoclave.

Le traitement selon l'Exemple 1 permet d'isoler 4,8 g de bromhydrate de N -/‾(n-pentyl)-1 éthylidène_7 N' - (imidazolino-2) hydrazine fondant à 110° C - Pureté (dosage perchlorique) : 100,7 %. $R^{dt}$ = 35 %.

EXEMPLE 16 :

N - /‾(bromo-4' phényl)-1 éthylidène_7 N' - (imidazolino-2) hydrazine, et acétate correspondant.

Formule (I) : $R_1$ = bromo-4 phényl
$R_2$ = méthyl
n = O

a) Mode opératoire identique à celui décrit dans l'Exemple 1 mais à partir de 19,9 g de bromo-4' phényl méthyl cétone (0,1 mole), 18,1 g de bromhydrate d'hydrazino-2 imidazoline-2 (0,1 mole) dans 200 ml d'éthanol pendant 6 heures au reflux.

On isole ainsi 25,4 g de bromhydrate de N - /‾(bromo-4' phényl)-1 éthylidène_7 N' - (imidazolino-2) hydrazine brut ($R^{dt}$ = 70 %), fondant à une température supérieure à 250° C.

b) Préparation de la base :

Le traitement à l'ammoniaque selon l'Exemple 1 de 12,5 g de bromhydrate précédemment isolé, donne 8 g de N - /‾(bromo-4' phényl)-1 éthylidène_7N' - (imidazolino-2) hydrazine fondant à 234-236° C.

Dosage du brome : théorie 28,4 %
trouvé 28,05 et 28,02 %

$R^{dt}$ à partir du bromhydrate : 83 %

c) Préparation de l'acétate :

7 g de la base précédemment isolée, en suspension dans 50 ml d'acétone sont traités sous agitation par 5 ml d'acide acétique. Tout passe en solution, puis un précipité apparaît. On agite encore 30 mn et on dilue avec 50 ml d'éther. Le précipité obtenu est essoré, rincé à l'éther, puis séché.

On obtient ainsi 6,8 g d'acétate de N - /(bromo-4' phényl)-1 éthylidène/ N' -(imidazolino-2) hydrazine ($R^{dt}$ = 80 %) fondant à 238° C - Pureté (dosage perchlorique): 99,04 % et 99,21 %.

EXEMPLE 17 :

N - /(chloro-4' phényl)-1 éthylidène/ N' -(imidazolino-2) hydrazine, et acétate correspondant.

Formule (I) : $R_1$ = chloro-4 phényl
$R_2$ = méthyl
n = O

a) Mode opératoire identique à celui décrit dans l'Exemple 1 mais à partir de 15,4 g de chloro-4' phényl méthyl cétone (0,1 mole), 18,1 g de bromhydrate d'hydrazino-2 imidazoline-2 (0,1 mole) dans 200 ml d'éthanol, pendant 6 heures au reflux.

On isole 20,3 g de bromhydrate de N - /(chloro-4' phényl)-1 éthylidène/ N' - (imidazolino-2) hydrazine brut ($R^{dt}$ 64 %) fondant à une température supérieure à 250° C.

b) Préparation de la base :

Le traitement à l'ammoniaque suivant l'Exemple 1 de 10 g de bromhydrate précédemment isolé, nous donne 6,2 g de N-/(chloro-4' phényl)-1 éthylidène/ N' - (imidazolino-2) hydrazine fondant à 214° C.

Dosage du chlore : théorie 14,98 %

trouvé 14,97 et 15,07 %

$R^{dt}$ à partir du bromhydrate : 83 %.

c) Préparation de l'acétate :

6 g de base précédemment isolée, en suspension dans 50 ml d'acétone, sont traités par 5 ml d'acide acétique. Après solubilisation partielle, le produit précipite. L'agitation est maintenue encore 30 min. Le précipité est essoré, et séché.

On obtient ainsi 4,3 g d'acétate de N - /(chloro-4' phényl)-1 éthylidène/ N'-(imidazolino-2) hydrazine ($R^{dt}$ = 57 %) fondant à 217° C - Pureté (dosage perchlorique) : 100,5 et 101,3 %.

EXEMPLE 18 :

N - /(tertiobutyl-4') cyclohexylidène/ N' - (imidazolino-2) hydrazine, et bromhydrate correspondant.

Formule (I) : $(R_1 R_2)$ = tertiobutyl-4 cyclohexyle

n = O

a) Mode opératoire identique à celui décrit dans l'Exemple 1 mais à partir de 6,16 g de tertiobutyl-4 cyclohexanone (0,04 mole), 7,2 g de bromhydrate d'hydrazino-2 imidazoline (0,04 mole) dans 90 ml d'éthanol et 20 ml d'eau, au reflux pendant 6 heures.

On obtient ainsi 12 g de bromhydrate de N - /(tertiobutyl-4') cyclohexylidène/ N' - (imidazolino-2) hydrazine fondant à 180-181° C ($R^{dt}$ = 94 %).

Dosage du brome : calculé 25,23 %
trouvé 25,0 et 24,9 %

b) Préparation de la base :

Le traitement à l'ammoniaque selon l'Exemple 1 de 8 g du bromhydrate précédemment isolé, nous donne 4,5 g de N - /(tertiobutyl-4') cyclohexylidène/ N' - (imidazolino-2) hydrazine fondant à 180° C - Dosage perchlorique : 100,9 et 101,3 %.

EXEMPLE 19 :

N - /(n-propyl-4' phényl)-1 éthylidène/ N' - (imidazolino-2) hydrazine et bromhydrate correspondant.

Formule I : $R_1$ = n-propyl-4 phényl
$R_2$ = méthyl
n = 0

a) Mode opératoire identique à celui décrit dans l'Exemple 1 mais à partir de 6,5 g de n-propyl-4' phényl méthyl cétone (0,04 mole), 7,25 g de bromhydrate d'hydrazino-2 imidazoline (0,04 mole) dans 80 ml de butanol, au reflux pendant 9 heures.

On obtient ainsi 10 g de bromhydrate de N - /(n-propyl-4' phényl)-1 éthylidène/ N' - (imidazolino-2) hydrazine, ($R^{dt}$ = 75 %) fondant à une température supérieure à 250° C.
F (appareil Totoli) : 259-60° C.

Dosage du brome : calculé 24,6 %
trouvé 24,1 et 24,5 %.

b) Préparation de la base :

Le traitement à l'ammoniaque selon l'Exemple 1 de 4,9 g du bromhydrate précédemment isolé nous permet d'obtenir 3,4 g de N - /(n-propyl-4' phényl)-1 éthylidène/

N' - (imidazolino-2) hydrazine fondant à 180° C - Pureté (dosage perchlorique) : 99,4 et 99,9 % - $R^{dt}$ 92 %.

EXEMPLE 20 :

N- /‾(trifluoro méthyl-3' phényl)-1 éthylidène/ N'- (imidazolino-2) hydrazine, et bromhydrate correspondant.

Formule I : $R_1$ = trifluoro méthyl-3 phényl
$R_2$ = méthyl
n = O

a) Mode opératoire identique à celui décrit dans l'Exemple 1 mais à partir de 10 g de trifluoro méthyl-3' phényl méthyl cétone (0,053 mole), 9,6 g de bromhydrate d'hydrazino-2 imidazoline (0,053 mole) dans 100 ml de butanol, au reflux pendant 6 heures.

On isole ainsi 10,3 g de bromhydrate de N - /‾(trifluoro méthyl-3' phényl)-1 éthylidène) N' - (imidazolino-2) hydrazine, ($R^{dt}$ = 55 %) fondant à 253° - 255°C.

Dosage du brome : théorie 22,75 %
trouvé 22,5 %

b) Préparation de la base :

Le traitement à l'ammoniaque selon l'Exemple 1 de 6 g du bromhydrate précédemment préparé nous permet d'isoler 4,2 g de N-/‾(trifluoro méthyl-3' phényl)-1 éthylidène/ N' - (imidazolino-2) hydrazine fondant à 188° C Pureté (dosage perchlorique) : 99,9 et 99,2 %. $R^{dt}$ = 91 %.

EXEMPLE 21 :

N - /‾(éthyl-5' cyclopenta (b) thiényl-2')-1 éthylidène/ N' - (imidazolino-2) hydrazine, et bromhydrate correspondant.

Formule I : $R_1$ = éthyl-5 cyclo penta (b) thiényl-2
$R_2$ = méthyl
n = O

a) Mode opératoire identique à celui décrit dans l'Exemple 1 mais à partir de 7,76 g d'(éthyl-5 cyclopenta (b) thiényl-2) méthyl cétone (0,04 mole), 7,2 g de bromhydrate d'hydrazino-2 imidazoline dans 80 ml d'éthanol et 20 ml d'eau au reflux pendant 8 heures.

On obtient 8,8 g de bromhydrate de N - /‾(éthyl-5' cyclopenta (b) thiényl-2')-1 éthylidène/ N' - (imidazolino-2) hydrazine ($R^{dt}$ = 80 %) fondant à une température supérieure à 250° C. F (appareil Totoli) : 290° C.

Dosage microanalyse (CHNSBr) : conforme.

b) Préparation de la base :

Le traitement à l'ammoniaque selon l'Exemple 1 de 6,8 g du bromhydrate précédemment isolé nous donne 5 g de N - /‾(éthyl-5' cyclopenta (b) thiényl-2')-1 éthylidène/ N ' - (imidazolino-2) hydrazine fondant à 236° C. $R^{dt}$= 95 %.

Dosage microanalyse (CHNS) : conforme.

EXEMPLE 22 :

N - /‾(phénoxy-3' phényl)-1 éthylidène/
N ' - (imidazolino-2) hydrazine et chlorhydrate correspondant.

Formule I : $R_1$ = phénoxy-3 phényl
$R_2$ = méthyl
n = 0

a) Mode opératoire identique à celui décrit dans l'Exemple 1 mais à partir de 6,7 g de phénoxy-3' phényl méthyl cétone (0,0316 mole), 5,7 g de bromhydrate d'hydrazino-2 imidazoline (0,0315 mole) dans 100 ml de butanol, au reflux pendant 9 heures.

On isole 7,30 g de bromhydrate de N - /‾(phénoxy-3' phényl)-1 éthylidène/ N' -imidazolino-2) hydrazine brut attendu ($R^{dt}$ = 60 %), fondant vers 230° C.

b) Préparation de la base :

Le traitement à l'ammoniaque, selon l'Exemple 1, de 7,30 g du bromhydrate précédemment préparé, nous permet d'obtenir 5,73 g de N - /(phénoxy-3' phényl)-1 éthylidène/ N ' - (imidazolino-2) hydrazine fondant à 160° C. Pureté (dosage perchlorique) : 98,5 et 98 % - $R^{dt}$ : 91 %.

c) Préparation du chlorhydrate :

2 g de base précédemment isolée en solution dans l'éther sont traités par de l'éther chlorhydrique jusqu'à acidité. Le précipité obtenu est essoré, rincé à l'éther et séché.

On obtient 2 g de chlorhydrate de N - /(phénoxy-3' phényl)-1 éthylidène/ N' - (imidazolino-2) hydrazine fondant à 240° C.

Dosage chlore ionisable : calculé 10,7 %
trouvé 10,6 et 10,8 %

$R^{dt}$ : 89 %.

EXEMPLE 23 :

N - /(isopentyl-4' phényl)-1 éthylidène/
N' - (imidazolino-2) hydrazine.

Formule I : $R_1$ = isopentyl-4 phényl
$R_2$ = méthyl
n = 0

Mode expérimental identique à celui décrit dans l'Exemple 1 mais à partir de 5 g d'isopentyl-4' phényl méthyl cétone (0,0263 mole) 4,80 g de bromhydrate d'hydrazino-2 imidazoline (0,0264 mole), dans 50 ml de butanol-1, au reflux pendant 9 heures.

On obtient ainsi 7,4 g du bromhydrate de N - /(isopentyl-4' phényl)-1 éthylidène/ N'- (imidazolino-2) hydrazine brut ($R^{dt}$ = 79,6 %) fondant vers 250° C.

Le traitement à l'ammoniaque selon l'Exemple 1 de 7,6 g du bromhydrate précédemment isolé, nous donne 4,5 g de N - /¯(isopentyl-4' phényl)-1 éthylidène/ N' - (imidazolino-2)hydrazine, fondant à 160° C - Pureté (dosage perchlorique) : 98,9 %.

$R^{dt}$ à partir du bromhydrate : 76 %

$R^{dt}$global : 60 %.

EXEMPLE 24 :

N - /¯(isopropyl-2' indanyl-5') éthylidène/ N' - (diméthyl 5,5 tétrahydro - 3,4,5,6 pyrimidino-2) hydrazine, et bromhydrate correspondant.

Formule I : $R_1$ = isopropyl-2 indanyl-5
$R_2$ = méthyl
n = 1 avec $R_3$ = $R_4$ = méthyl

a) Mode expérimental identique à celui décrit dans l'Exemple 1 mais à partir de 7 g d'isopropyl-2 acétyl-5 indane (0,0348 mole) et 7,8 g de bromhydrate d'hydrazino-2 diméthyl-5,5 tétrahydro-3,4,5,6 pyrimidine-2 (0,0348 mole) en solution dans 50 ml de butanol, au reflux pendant 6 heures.

On isole ainsi 11,4 g de bromhydrate de N - /¯(isopropyl-2' indanyl-5')-1 éthylidène/ N' - (diméthyl-5, 5 tétrahydro - 3,4,5,6 pyrimidino-2) hydrazine ($R^{dt}$ = 80 %) dont le point de fusion est supérieur à 250° C.

Dosage perchlorique : 100,7 %.

F (appareil Totoli) : 275-6° C.

b) Préparation de la base :

7,6 g du bromhydrate précédent en solution dans le chloroforme sont traités pendant 3 heures, par une solution d'ammoniaque.

La solution est ensuite lavée à l'eau, séchée et évaporée à sec.

On obtient 3,8 g de N -/ (isopropyl-2' indanyl-5')-1 éthylidène/ N' - (diméthyl-5,5 tétrahydro-3,4,5,6 pyrimidino-2) hydrazine ($R^{dt}$ : 62 %) fondant à 195° C - Pureté (dosage perchlorique) : 99 %.

EXEMPLE 25 :

N - / (tertio butyl-4' phényl)-1 éthylidène/ N'-(diméthyl-5,5 tétrahydro-3,4,5,6 pyrimidino-2) hydrazine et bromhydrate correspondant.

Formule I : $R_1$ = tertio butyl-4 phényl
$R_2$ = méthyl
n = 1 avec $R_3$ = $R_4$ = méthyl

a) Mode expérimental identique à celui décrit dans l'Exemple 1 mais à partir de 6,1 g de tertio butyl-4' phényl méthyl cétone (0,0348 mole) et 7,8 g de bromhydrate d'hydrazino-2 diméthyl-5,5 tétrahydro-3,4,5,6 pyrimidine-2 (0,0348 mole) dans 50 ml de butanol au reflux pendant 6 heures.

On isole ainsi 10 g de bromhydrate de N - / (tertiobutyl-4' phényl)-1 éthylidène/ N' - (diméthyl-5,5 tétrahydro-3,4,5,6 pyrimidino-2) hydrazine ($R^{dt}$ = 75 %) fondant à une température supérieure à 260° C - Pureté (dosage perchlorique) : 100 %. F (appareil Totoli) : 285-6° C.

b) Préparation de la base :

Le traitement à l'ammoniaque selon l'Exemple 1 de 7 g du bromhydrate précédent donne 5,3 g de N - / (tertiobutyl-4' phényl)-1 éthylidène/ N'-(diméthyl-5,5 tétrahydro -3,4,5,6 pyrimidino-2) hydrazine ($R^{dt}$ : 94 %). Fondant à 210-212° C - Pureté (dosage perchlorique) : 100 %.

EXEMPLE 26 :

Bromhydrate de N- /(phényl-1 éthylidène)/ N' - (imidazolino-2) hydrazine.

Formule I : $R_1$ = phényl
$R_2$ = méthyl
n = O

Mode opératoire identique à celui décrit dans l'Exemple 1, mais à partir de 10 g de phényl méthyl cétone (0,085 mole) et 15 g de bromhydrate d'hydrazino-2 imidazoline (0,082 mole) dans 100 ml de butanol, au reflux pendant 6 heures.

On isole ainsi 20 g de bromhydrate de N-/(phényl-1 éthylidène)/ N'-(imidazolino-2) hydrazine ($R^{dt}$ : 83 %) fondant à une température supérieure à 250 ° C - Pureté (dosage perchlorique): 100,5 %. F (appareil Totoli) : 254-6° C.

EXEMPLE 27 :

Bromhydrate de N - /(méthylthio-2' pyridyl-3')-1 méthylène/ N' - (imidazolino-2) hydrazine.

Formule I : $R_1$ = méthylthio-2 pyridyl-3
$R_2$ = H
n = O

Une solution de 13,4 g de méthylthio-2 pyridyl-3 carboxaldéhyde (0,0873 mole) et 15,8 g de bromhydrate d'hydrazino-2 imidazoline (0,0873 mole) dans 160 ml d'éthanol et 100 ml d'eau, est portée au reflux pendant 12 h 30.

La solution est évaporée à sec. Le résidu est recristallisé dans l'éthanol.

On obtient ainsi 12,5 g de bromhydrate de N-/(méthylthio-2' pyridyl-3')-1 méthylène/ N' - (imidazolino-2) hydrazine ($R^{dt}$ = 46 %) fondant à 250° C.

Dosage du brome : trouvé 25 %
théorie 25,3 %.

EXEMPLE 28 :

Phosphate de N - /(isopropyl-2' indanyl-5')-1 éthylidène/ N'-(imidazolino-2) hydrazine.

Formule I : $R_1$ = isopropyl-2 indanyl-5
$R_2$ = méthyl
n = O

4 g de N-/(isopropyl-2' indanyl-5') éthylidène/ N'-(imidazolino-2) hydrazine (0,014 mole), préparé dans l'Exemple 2, sont mis en suspension dans 50 ml de méthanol et sont traités par 1 ml d'acide orthophosphorique à 85 % (d = 1,7) pendant 2 heures sous agitation à température ambiante.

Le précipité est essoré, lavé au méthanol puis à l'éther et séché.

On obtient ainsi 4,4 g de phosphate de N-/(isopropyl-2' indanyl-5')-1 éthylidène/ N' - (imidazolino-2) hydrazine, ($R^{dt}$ = 82%) fondant à 266° C (appareil Totoli) - Pureté (dosage perchlorique) : 100,3 %.

EXEMPLE 29 :

Bromhydrate de N-/(méthylthio-2' pyridyl-3')-1 éthylidène/ N'-(diméthyl-5,5 tétrahydro-3,4,5, 6 pyrimidino-2) hydrazine.

Formule I : $R_1$ = méthylthio-2 pyridyl-3
$R_2$ = méthyl
n = 1 avec $R_3$ = $R_4$ = méthyl

Mode opératoire identique à celui décrit dans l'Exemple 1 mais à partir de 8,4 g de (méthylthio-2 pyridyl-3) méthyl cétone (0,05 mole), et 11,2 g de bromhydrate d'hydrazino-2 diméthyl-5,5 tétrahydro-3,4,5, 6 pyrimidine-2 (0,05 mole) dans 75 ml de butanol, au

reflux pendant 8 heures.

Après refroidissement, la solution est étendue à l'éther.

Le précipité obtenu est filtré, lavé à l'éther, puis repris dans 50 ml d'eau et mis sous agitation pendant 1 heure.

Le produit obtenu est essoré et séché.

On obtient ainsi 4,3 g de bromhydrate de N - /¯(méthylthio-2' pyridyl-3')-1 éthylidène/ N' - (diméthyl-5,5 tétrahydro-3,4,5,6 pyrimidino-2) hydrazine, fondant à 236° C ($R^{dt}$ = 23 %) - Pureté (dosage perchlorique) : 100 %.

EXEMPLE 30 :

N-/¯(méthylthio-2' pyridyl-3')-1 éthylidène/ N' - (imidazolino-2) hydrazine et bromhydrate correspondant.

Formule I : $R_1$ = méthylthio-2 pyridyl-3
$R_2$ = méthyl
n = 0

a) Mode opératoire identique à celui décrit dans l'Exemple 1 mais à partir de 10 g de (méthylthio-2 pyridyl-3) méthyl cétone (0,06 mole) et 10,8 g de bromhydrate d'hydrazino-2 imidazoline (0,06 mole) dans 100 ml de butanol au reflux pendant 9 heures.

Le produit précipite en cours de réaction.

Après refroidissement, le précipité est filtré, lavé et séché.

On obtient ainsi 15 g de bromhydrate de N - /¯(méthylthio-2' pyridyl-3')-1 éthylidène/ N' - (imidazolino-2) hydrazine ($R^{dt}$ = 76 %) fondant à 228° C (décomposition).

b) Préparation de la base :

Le traitement à l'ammoniaque de 7,5 g du bromhydrate précédemment préparé permet d'obtenir 4,3 g de N - /(méthylthio-2' pyridyl-3')-1 éthylidène/ N' - (imidazolino-2) hydrazine fondant à 218° C. ($R^{dt}$ = 75 %).

EXEMPLE 31 :

Action des composés sur la croissance mycélienne.

Le milieu nutritif utilisé est le milieu P.D.A. de composition suivante :

- pomme de terre : 200 g
- glucose : 20 g
- agar-agar : 16 g
- eau q.s.p. : 1000 ml

Des suspensions aqueuses diluées des composés à tester sont incorporées dans ce milieu maintenu en fusion à 45° C, à raison d'une partie en volume pour 9 parties en volume de milieu nutritif. Les dilutions sont telles que les concentrations finales en composé dans le milieu nutritif traité sont les suivantes :

$C_0$ = 0 ppm (témoin)

$C_4$ = 100 ppm

Le milieu est ensuite coulé dans des boîtes de Pétri de 90 mm de diamètre où on le laisse refroidir et solidifier. Chaque boîte est contaminée à l'aide de fragments de mycélium prélevés dans des cultures de champignons agées de huit jours. Ces champignons sont Fusarium roseum, Rhizoctonia solani, Phomopsis viticola, Alternaria tenuis et Botrytis fabae.

On met les boîtes à incuber pendant 2 jours dans une chambre maintenue à 22° C et à un taux d'hygrométrie de 70 %. Après ces deux jours, le mycélium s'est développé, réalisant autour des points de contamination des plages mycéliennes circulaires dont on mesure le diamètre. Le diamètre moyen des plages mycéliennes obtenues dans les boîtes contenant le milieu nutritif traité avec les composés à tester est comparé au diamètre moyen des plages mycéliennes obtenues dans les boîtes contenant le milieu nutritif non traité (témoin).

Les résultats sont exprimés selon une échelle de 0 à 4 :

0 - le diamètre de la colonie traitée est identique à celui de la colonie témoin.

1 - l'inhibition de croissance mycélienne est faible.

2 - l'inhibition de croissance mycélienne est moyenne.

3 - l'inhibition de croissance mycélienne est forte.

4 - l'inhibition de croissance est totale, le mycélium ne s'est pas développé du tout.

Les résultats obtenus sont présentés dans le tableau I ci-après.

/ Dans les tableaux I à VIII ci-après, les composés utilisés dans les exemples d'applications fongicides seront désignés de la manière suivante :

- une base : chiffre romain seul qui correspond à l'exemple de préparation chimique ;
- la forme acétate : chiffre romain affecté de l'indice "a" ;
- la forme bromhydrate : chiffre romain affecté de l'indice "b" ;
- la forme chlorhydrate : chiffre romain affecté de l'indice "c" ;

- la forme maléate : chiffre romain affecté de l'indice "m".]

EXEMPLE 32 :

Action des composés à la concentration de 100 ppm sur la germination des spores.

On utilise le même milieu nutritif que dans l'exemple 31, et on y incorpore les composés à tester de la même façon. La concentration finale en composé dans le milieu nutritif traité est la suivante :

. $C_4$ = 100 ppm

Le milieu ainsi traité est coulé dans les alvéoles de plaques de Ciréra et on le laisse refroidir et solidifier. Le milieu nutritif solidifié est contaminé en déposant dans chaque alvéole 50 µl d'une suspension aqueuse de spores de l'un des champignons suivants : Botrytis cinerea, Alternaria tenuis, Penicillium expansum, Aspergillus niger.

Après incubation pendant 24 heures à la température de 22° C on compte le nombre de spores non germées et on compare ce nombre au nombre total de spores. Les résultats sont finalement exprimés selon une échelle : 0 - 2 - 4 où :

0 - représente une inhibition de la germination nulle,

2 - représente une inhibition de la germination moyenne,

4 - représente une inhibition de la germination totale.

Les résultats sont rassemblés dans le tableau II ci-après.

EXEMPLE 33 :

Action de quelques composés sur la germination des spores de Botrytis cinerea en fonction de la concentration.

On utilise le même milieu nutritif que dans l'exemple précédent et on y incorpore les composés à tester de la même façon. Les concentrations finales en composés dans le milieu nutritif traité sont les suivants :

- $C_1$ = 1,56 ppm
- $C_2$ = 6,25 ppm
- $C_3$ = 25 ppm
- $C_4$ = 100 ppm

Le milieu ainsi traité est coulé dans les alvéoles de plaques de Ciréra et on le laisse refroidir et solidifier. Le milieu nutritif solidifié est contaminé en déposant dans chaque alvéole 50 µl d'une suspension aqueuse de spores du champignon suivant : Botrytis cinerea.

Après incubation pendant 24 heures à la température de 22° C, on compte le nombre de spores non germées et on compare ce nombre au nombre total de spores. Les résultats sont finalement exprimés par un chiffre de 0 à 100 qui représente le pourcentage d'inhibition de la germination des spores. 0 signifie donc que toutes les spores ont germé, 100 qu'aucune spore n'a germé.

Les résultats sont rassemblés dans le tableau III ci-après.

EXEMPLE 34 :

Action préventive des composés vis-à-vis de la rouille du haricot.

On prépare des suspensions aqueuses des composés

à tester, de concentrations suivantes :

. $C_0$ = 0 (témoin)

. $C_4$ = 1000 ppm

Ces suspensions sont pulvérisées sur des plants de haricot "Mange tout plein le panier", cultivés en pots et âgés de 12 jours. La quantité de suspension apportée par unité de surface équivaut à 1000 l/ha. 24 heures après le traitement, on pulvérise une suspension aqueuse de spores d'Uromyces phaseoli sur le feuillage des haricots, puis on met les plantes en atmosphère saturée d'eau, à la température de 22° C, pendant 48 heures. Les plantes sont ensuite placées dans une salle maintenue à la température de 22° C et à un taux d'hygrométrie de 80 %.

12 jours après le traitement, on note le nombre de taches de rouille présentes sur les feuilles, d'une part dans le cas des plantes traitées à l'aide des composés à tester, d'autre part dans le cas des plantes témoins. On en déduit le niveau d'action du composé sur la maladie.

La notation se fait selon une échelle de 0 à 4 :

- 0 = pas d'effet du composé,
- 1 = action faible du composé,
- 2 = action moyenne du composé,
- 3 = action forte du composé,
- 4 = action totale du composé.

Les résultats sont rassemblés dans le tableau IV ci-après.

<u>EXEMPLE 35</u> :

<u>Action préventive des composés vis-à-vis de la rouille du haricot</u>.

On prépare des suspensions aqueuses des composés

à tester, de concentrations suivantes :

. $C_0$ = 0 (témoin)

. $C_1$ = 15,6 ppm

. $C_2$ = 62,5 ppm

. $C_3$ = 250 ppm

. $C_4$ = 1000 ppm

Le protocole est pour la suite identique à celui de l'exemple 34, à l'exception de la notation où on apprécie l'efficacité du traitement : 0 signifie qu'il y a un développement de la maladie identique à celui des plantes non traitées ; 100 indique que la maladie n'apparaît pas (efficacité maximum).

Les résultats sont rassemblés dans le tableau V ci-après.

EXEMPLE 36 :

Action préventive des composés vis-à-vis de l'Oïdium de l'orge.

On prépare des suspensions aqueuses du composé à tester selon une échelle de concentration identique à celle de l'exemple 35, puis on pulvérise ces suspensions sur des plants d'orge "Rika" agés de 12 jours. Ces suspensions sont pulvérisées à raison de 1000 l/ha. 24 heures après le traitement, on saupoudre sur le feuillage des orges des spores d'Erysiphe graminis et on laisse les plantes en serre à 22° C pendant 7 jours. Au bout de ce laps de temps, on note le nombre de taches d'oïdium présentes sur la première feuille, d'une part dans le cas des plantes traitées à l'aide du composé à tester, d'autre part dans le cas des plantes témoins non traitées. On en déduit l'efficacité du traitement : 0 signifie qu'il y a un développement de la maladie identique à celui des

plantes non traitées ; 100 indique que la maladie n'apparaît pas (efficacité maximum).

Les résultats sont rassemblés dans le tableau VI ci-après.

EXEMPLE 37 :

Action préventive des composés sur le Botrytis de la fève.

On prépare des suspensions aqueuses des composés à tester selon la même échelle de concentrations que dans l'exemple 35. Ces suspensions sont pulvérisées à raison de 1000 l/ha sur des plants de fève agés de 10 jours. Après quoi, des suspensions des spores de Botrytis fabae servent à contaminer les fèves déjà traitées. Les plantes restent en humidité saturante pendant 24 heures, puis sont entreposées en salle climatisée à 22° C et 70 % d'humidité. Après 5 jours, on effectue la notation selon une échelle de 0 à 100 ; 0 indiquant qu'il y a un développement de la maladie identique à celui des plantes non traitées. 100 indiquant que la maladie n'apparaît pas.

Les résultats sont présentés dans le tableau VII ci-après.

EXEMPLE 38 :

Action préventive des composés sur Mildiou de la tomate.

On prépare des suspensions aqueuses des composés à tester selon la même échelle de concentration que dans l'exemple 35. Ces suspensions sont pulvérisées à raison de 1000 l/ha sur des plants de tomates agés de 20 jours. Ces plantes sont contaminées à l'aide d'une suspension de spores de Phytophtora infestans et mises en humidité saturante pendant 24 heures.

Les plantes sont ensuite entreposées en salle climatée à 22° C et 70 % d'humidité. Après 7 jours, on effectue la notation selon l'échelle de 0 à 100 ; le 0 indiquant qu'il y a développement de la maladie identique à celui des plantes non traitées ; 100 signifiant que la maladie n'apparaît pas.

Les résultats sont présentés dans le tableau VIII ci-après.

EXEMPLE I' : N-[(n-pentyl-4' phényl)-1 éthylidène] N'-(imidazolino-2) hydrazine et bromhydrate correspondant.

Formule I' : $R_1$ = n-pentyl-4 phényl

Mode opératoire identique à celui décrit dans l'exemple 1, mais à partir de 14,3 g de n-pentyl-4' phényl méthyl cétone (0,075 mole), 13,6 g de bromhydrate d'hydrazino-2 imidazoline (0,075 mole) dans 100 ml de butanol, au reflux pendant 6 heures.

On obtient ainsi, après recristallisation dans le méthanol, 17,8 g de bromhydrate de N-[(n-pentyl-4' phényl) -1 éthylidène] N'-(imidazolino-2) hydrazine (Rendement = 67 %) fondant à 242-244° C.

Pureté (dosage perchlorique) : 100 et 100,6 %.

Préparation de la base :

Le traitement à l'ammoniaque selon l'Exemple 1 de 7 g de bromhydrate précédemment isolé permet d'obtenir 4,1 g de N-[(n-pentyl-4' phényl)-1 éthylidène] N'-(imidazolino-2) hydrazine fondant à 151° C.

Pureté (dosage perchlorique) : 99,8 % et 99,7 %.

Rendement : 76 %.

EXEMPLE II' : N-[(n octyl-4' phényl)-1 éthylidène] N'-(imidazolino-2) hydrazine et bromhydrate correspondant.

Formule I' : $R_1$ = n octyl-4 phényl

Mode opératoire identique à celui décrit dans l'Exemple 1, mais à partir de 10,2 g de n octyl-4' phényl

méthyl cétone (0,044 mole), 7,9 g de bromhydrate d'hydrazino-2 imidazoline (0,044 mole) dans 100 ml de butanol, au reflux pendant 6 heures.

On obtient ainsi 10,1 g de bromhydrate de N-[(n octyl-4' phényl)-1 éthylidène] N'-(imidazolino-2) hydrazine (rendement = 58 %) fondant à 215°C.

Pureté (dosage perchlorique) : 99,8 % et 100 %.

Préparation de la base :

Le traitement à l'ammoniaque selon l'Exemple 1 de 5,5 g de bromhydrate précédemment isolé permet d'obtenir 3,7 g de N-[(n octyl-4' phényl)-1 éthylidène] N'-(imidazolino-2) hydrazine fondant à 130° C.

Pureté (dosage perchlorique) : 98,9 et 99 %.

Rendement : 84 %.

EXEMPLE III' : N-[(n hexyl-4' phényl)-1 éthylidène] N'(imidazolino-2)hydrazine et bromhydrate correspondant.

Formule I' : $R_1$ = n hexyl-4 phényl

Mode opératoire identique à celui décrit dans l'Exemple 1 mais à partir de 11 g de n-hexyl-4' phényl méthyl cétone (0,054 mole), 9,8 g de bromhydrate d'hydrazino-2 imidazoline (0,054 mole), dans 100 ml de butanol au reflux pendant 6 heures.

On obtient ainsi 17,2 g de bromhydrate de N-[(n hexyl-4' phényl)-1 éthylidène] N'-(imidazolino-2) hydrazine (rendement = 87 %) fondant à 212° C.

Pureté (dosage perchlorique) : 98,7% et 98 %.

Préparation de la base :

Le traitement à l'ammoniaque selon l'Exemple 1 de 10,5 g de bromhydrate précédemment isolé permet d'obtenir 7 g de N-[(n hexyl-4' phényl)-1 éthylidène] N' (imidazolino-2) hydrazine fondant à 143° C.

Pureté (dosage perchlorique) : 99,2 %.

Rendement : 87 %.

EXEMPLE IV' : N-[(n dodécyl-4' phényl)-1 éthylidène] N'-(imidazolino-2) hydrazine et bromhydrate correspondant.

Formule I' : $R_1$ = n dodécyl-4 phényl

Mode opératoire identique à celui décrit dans l'Exemple 1 à partir de 17,3 g de n dodécyl-4' phényl méthyl cétone (0,060 mole), 11 g de bromhydrate d'hydrazine-2 imidazoline (0,060 mole), dans 100 ml de butanol, au reflux pendant 6 heures.

On obtient ainsi 21,3 g de bromhydrate de N-[(n dodécyl-4' phényl)-1 éthylidène] N'-(imidazolino-2) hydrazine (rendement = 79 % fondant à 214°-215°C.

Pureté (dosage perchlorique) : 100,5 %.

Préparation de la base :

Le traitement à l'ammoniaque selon l'Exemple 1 de 11 g de bromhydrate précédemment isolé, permet d'obtenir 8,2 g de N-[(n dodécyl-4' phényl)-1 éthylidène] N'-(imidazolino-2) hydrazine, fondant à 126° C.

Pureté (dosage perchlorique) : 99,7 %.

Rendement : 92 %.

EXEMPLE V' : N-[(iso hexyl-4' phényl)- éthylidène] N'-(imidazolino-2) hydrazine et bromhydrate correspondant.

Formule I' : $R_1$ = iso hexyl-4 phényl

Mode opératoire identique à celui décrit dans l'Exemple 1, mais à partir de 12 g d'iso hexyl-4' phényl méthyl cétone (0,059 mole), 11 g de bromhydrate d'hydrazino-2 imidazoline (0,059 mole) dans 70 ml de butanol, au reflux pendant 6 heures.

On obtient ainsi, 18,8 g de bromhydrate de N[(iso hexyl-4' phényl)-1 éthylidène] N'-(imidazolino-2) hydrazine, fondant à 212°-213° C. Rendement = 87 %.

Pureté (dosage perchlorique) : 100,5 %.

Préparation de la base :

Le traitement à l'ammoniaque selon l'Exemple 1 de 10 g de bromhydrate précédemment isolé permet d'obtenir 7,3 g de N-[(iso hexyl-4' phényl)-1 éthylidène] N' (imidazolino-2) hydrazine fondant à 137°-138° C.

Pureté (dosage perchlorique) : 99,5 %.

Rendement : 92 %.

EXEMPLE VI' : N-/(iso heptyl-4' phényl)-1 éthylidène/ N'-(imidazolino-2) hydrazine et bromhydrate correspondant.

Formule I' : $R_1$ = iso heptyl-4 phényl

Mode opératoire identique à celui décrit dans l'Exemple 1 mais à partir de 14,2 g d'iso heptyl-4' phényl méthyl cétone (0,065 mole), 11,8 g de bromhydrate d'hydrazino-2 imidazoline (0,065 mole) dans 70 ml de butanol au reflux pendant 6 heures.

On obtient ainsi après recristallisation dans l'acétonitrile, 16,3 g de bromhydrate de N-/(iso heptyl-4' phényl)-1 éthylidène/ N'(imidazolino-2) hydrazine (rendement = 66 %) fondant à 212°-213° C.

Pureté (dosage perchlorique) : 99 %.

Préparation de la base :

Le traitement à l'ammoniaque selon l'Exemple 1 de 10 g de bromhydrate précédemment isolé permet d'obtenir 7,6 g de N-/(iso heptyl-4' phényl)-1 éthylidène/ N'-(imidazolino-2) hydrazine fondant à 136°-137° C.

Pureté (dosage perchlorique) : 99,3 %.

Rendement : 93 %.

Action préventive des composés vis-à-vis de la rouille du haricot :

On prépare des suspensions aqueuses des composés à tester, de concentrations suivantes :

. $C_0$ = 0 (témoin)

. $C_4$ = 1000 ppm

Ces suspensions sont pulvérisées sur des plants de haricots "Mange tout plein le panier", cultivés en pots et âgés de 12 jours. La quantité de suspension apportée par unité de surface équivaut à 1000 l/ha. 24 heures après le traitement, on pulvérise une suspension aqueuse de spores d'Uromyces phaseoli sur le feuillage des haricots, puis on met les plantes en atmosphère saturée d'eau, à la température de 22° C, pendant 48 heures. Les plantes sont ensuite

placées dans une salle maintenue à la température de 22° C et à un taux d'hygrométrie de 80 %.

12 jours après le traitement, on note le nombre de taches de rouille présentes sur les feuilles, d'une part dans le cas des plantes traitées à l'aide des composés à tester, d'autre part dans le cas des plantes témoins. On en déduit le niveau d'action du composé sur la maladie.

La notation se fait selon une échelle de 0 à 4 :

- 0 = pas d'effet du composé,
- 1 = action faible du composé,
- 2 = action moyenne du composé,
- 3 = action forte du composé,
- 4 = action totale du composé.

Les résultats sont rassemblés dans le Tableau IV' ci-après.

TABLEAU I

EFFET SUR LA CROISSANCE MYCELIENNE

notes de 0 à 4

| Champignons / Composé | Fusarium roseum | Phomopsis viticola | Rhizoctonia solani | Alternaria tenuis | Botrytis cineres |
|---|---|---|---|---|---|
| I | 3 | 4 | 2 | 3 | - |
| II | 3 | 4 | 2 | 3 | 2 |
| III | 3 | 4 | 2 | 3 | - |
| IVa | 2 | 4 | 2 | 2 | - |
| Va | 2 | 4 | 2 | 2 | - |
| VI | 2 | 4 | 1 | 2 | 4 |
| VIb | 2 | 4 | 1 | 2 | 3 |
| VII | 2 | 4 | 2 | 2 | 3 |
| VIIb | 3 | 4 | 2 | 2 | 3 |
| VIII | 3 | 4 | 2 | 3 | 4 |
| VIIIb | 3 | 2 | 2 | 3 | 2 |
| IX | 2 | 4 | 2 | 2 | 3 |
| IXc | 3 | 4 | 2 | 2 | 3 |
| X | 2 | 4 | 1 | 2 | 3 |
| Xc | 3 | 4 | 1 | 2 | 4 |
| XI | 3 | 2 | 1 | 2 | 1 |
| XIIb | 3 | 3 | 1 | 2 | 1 |

TABLEAU II

EFFET SUR LA GERMINATION DES SPORES

| Champignons / Composé | Botrytis cinerea | Alternaria tenuis | Penicillium expansum | Aspergillus niger |
|---|---|---|---|---|
| I | 4 | 4 | 0 | 2 |
| II | 4 | 4 | 2 | 2 |
| III | 4 | 2 | 0 | 0 |
| IVa | 2 | 0 | 0 | 0 |
| Va | 2 | 0 | 0 | 0 |
| VI | 4 | 2 | 0 | 0 |
| VIb | 2 | 2 | 0 | 0 |
| VII | 4 | 2 | 0 | 2 |
| VIIb | 4 | 2 | 0 | 0 |
| VIII | 4 | 4 | 2 | 2 |
| VIIIb | 4 | 2 | 0 | 0 |
| IX | 2 | 2 | 0 | 0 |
| IXc | 2 | 2 | 0 | 0 |
| X | 4 | 2 | 0 | 0 |
| Xc | 4 | 2 | 0 | 0 |
| XIIb | 2 | 2 | 0 | 0 |

TABLEAU III

POUVOIR INHIBANT DE LA GERMINATION DES SPORES SUR BOTRYTIS CINEREA

| Concentration ppm / Composé | $C_1$ | $C_2$ | $C_3$ | $C_4$ |
|---|---|---|---|---|
| II | 6 | 18 | 83 | 100 |
| VI | 0 | 2 | 3 | 50 |
| VIII | 2 | 3 | 27 | 100 |
| VIIIb | 2 | 3 | 5 | 90 |
| X | - | 2 | 7 | 32 |

TABLEAU IV

ACTION PREVENTIVE SUR LA ROUILLE DU HARICOT

| Concentration / Composé | 1 000 ppm |
|---|---|
| I | 3 |
| II | 4 |
| III | 4 |
| IVa | 3 |
| Va | 3 |
| VI | 4 |
| VIb | 4 |
| VII | 4 |
| VIIb | 4 |
| VIII | 4 |
| VIIIb | 4 |
| IX | 4 |
| IXc | 4 |
| X | 4 |
| XI | 3 |
| XIIb | 4 |
| XIIIb | 1 |
| XIVb | 1 |
| XVb | 1 |
| XXVI | 2 |

# TABLEAU IV'

| Composés | | Concentration 1000 ppm |
|---|---|---|
| 1051-33 | EXEMPLE I' (base ou bromhydrate) | 4 |
| 1051-34 | EXEMPLE II' (base ou bromhydrate) | 4 |
| 1051-35 | EXEMPLE III' (base ou bromhydrate) | 4 |
| 1051-36 | EXEMPLE IV' base<br>EXEMPLE IV' bromhydrate | 2<br>1 |
| 1051-37 | EXEMPLE V' (base ou bromhydrate) | 4 |
| 1051-38 | EXEMPLE VI' (base ou bromhydrate) | 4 |

0062587

TABLEAU V

ACTION PREVENTIVE SUR ROUILLE DU HARICOT EN FONCTION DES CONCENTRATIONS

| Concentration / Composé | $C_1$ | $C_2$ | $C_3$ | $C_4$ |
|---|---|---|---|---|
| II | 17 | 38 | 100 | 100 |
| III | 15 | 50 | 100 | 100 |
| IVa | 15 | 20 | 96 | 100 |
| Va | 0 | 13 | 85 | 93 |
| VI | 8 | 15 | 77 | 100 |
| VIb | 30 | 30 | 77 | 100 |
| VII | 20 | 83 | 100 | 100 |
| VIIb | 18 | 73 | 100 | 100 |
| VIII | 15 | 86 | 100 | 100 |
| VIIIb | 17 | 86 | 100 | 100 |
| IX | 0 | 10 | 90 | 100 |
| IXc | 13 | 50 | 100 | 100 |
| X | 0 | 0 | 95 | 100 |
| Xc | 5 | 76 | 100 | 100 |
| XI | 0 | 18 | 46 | 80 |
| XIIb | 15 | 68 | 98 | 100 |

TABLEAU VI

Action sur Oïdium de l'orge en fonction de la concentration

| Concentration / Composé | $C_1$ | $C_2$ | $C_3$ | $C_4$ |
|---|---|---|---|---|
| II | 0 | 0 | 27 | 50 |
| VII | 9 | 10 | 31 | 41 |
| $XII_b$ | 2 | 28 | 31 | 67 |

TABLEAU VII

Action préventive sur Botrytis de la fève en fonction de la concentration.

| Concentration / Composé | $C_1$ | $C_2$ | $C_3$ | $C_4$ |
|---|---|---|---|---|
| II | 4 | 33 | 40 | 52 |
| VI | 0 | 0 | 40 | 60 |
| $VII_b$ | 0 | 0 | 0 | 40 |
| VIII | 0 | 0 | 40 | 50 |
| $VIII_b$ | 0 | 0 | 20 | 60 |
| $X_c$ | 0 | 0 | 0 | 20 |

TABLEAU VIII

Action préventive sur Mildiou de la tomate en fonction de la concentration.

| Concentration / Composé | $C_1$ | $C_2$ | $C_3$ | $C_4$ |
|---|---|---|---|---|
| VI | 0 | 13 | 30 | 100 |
| $VI_b$ | 0 | 13 | 69 | 100 |
| VII | 0 | 20 | 33 | 75 |
| $VII_b$ | 3 | 8 | 43 | 95 |
| VIII | 0 | 0 | 5 | 53 |
| $VIII_b$ | 0 | 13 | 75 | 98 |
| $XII_b$ | 0 | 5 | 18 | 63 |

TABLEAU IX

| Exemple | | | |
|---|---|---|---|
| 1 | 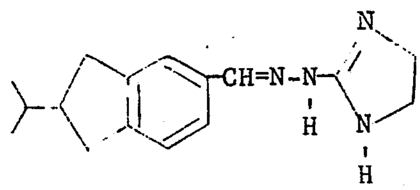 | base | 1051-02 |
| 2 | 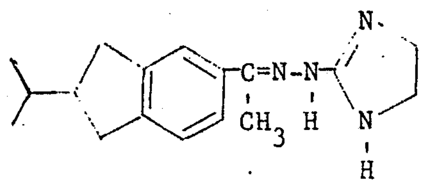 | base | 1051-03 |
| 3 | 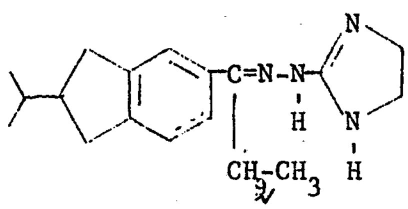 | base et maléate | 1051-05 |
| 4 | 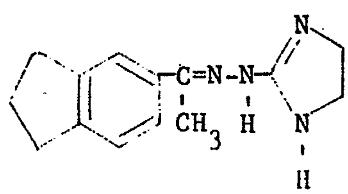 | acétate | 1051-07 |
| 5 | 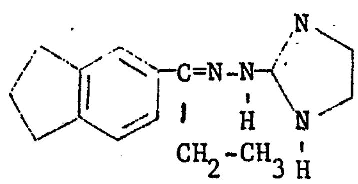 | acétate | 1051-08 |
| 6 | 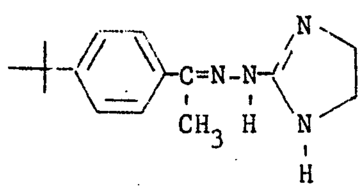 | base + bromhydrate | 1051-14 |
| 7 | 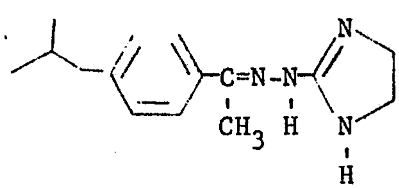 | base + bromhydrate | 1051-17 |

TABLEAU IX (suite 1)

| Exemple | | | |
|---|---|---|---|
| 8 | 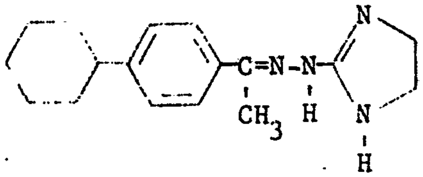 | base + bromhydrate | 1051-16 |
| 9 | 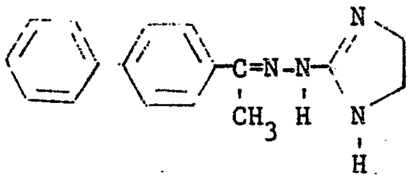 | base + chlorhydrate | 1051-13 |
| 10 | 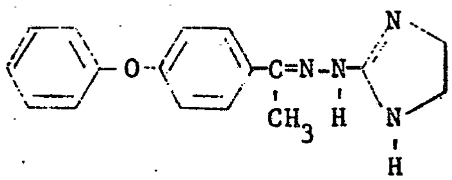 | base + chlorhydrate | 1051-12 |
| 11 | 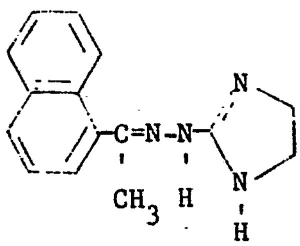 | base | 1051-15 |
| 12 | 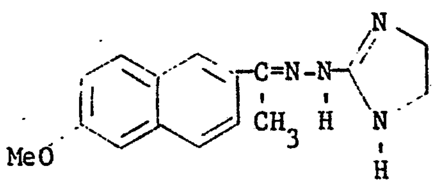 | bromhydrate | 1051-18 |
| 13 | 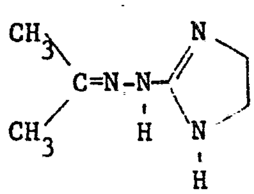 | bromhydrate | 1051-09 |
| 14 | 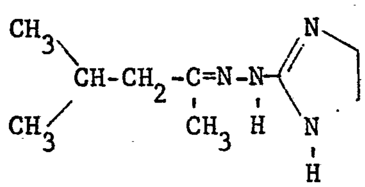 | bromhydrate | 1051-10 |

TABLEAU IX (suite 2)

| Exemple | | | |
|---|---|---|---|
| 15 | 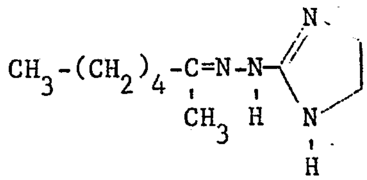 | bromhydrate | 1051-11 |
| 16 | 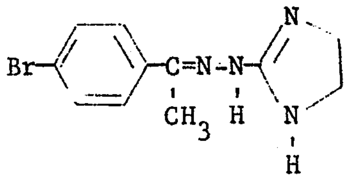 | base et acétate | 1051-19 |
| 17 | 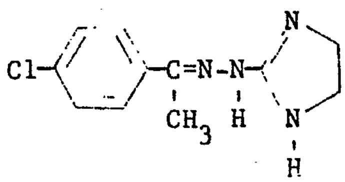 | base et acétate | 1051-20 |
| 18 | 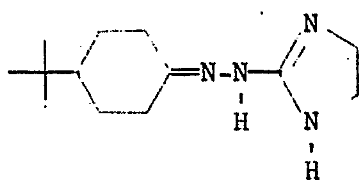 | base + bromhydrate | 1051-21 |
| 19 | 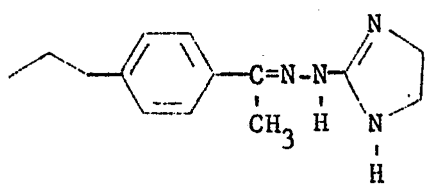 | base + bromhydrate | 1051-22 |
| 20 | 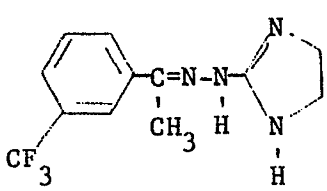 | base + bromhydrate | 1051-23 |
| 21 | 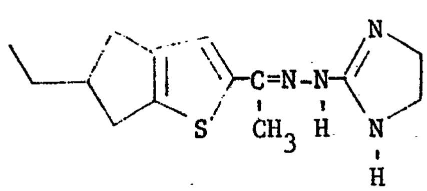 | base + bromhydrate | 1051-24 |

TABLEAU IX (suite 3)

| Exemple | | | |
|---|---|---|---|
| 22 | 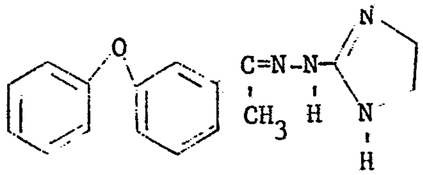 | base + chlorhydrate | 1051-25 |
| 23 | 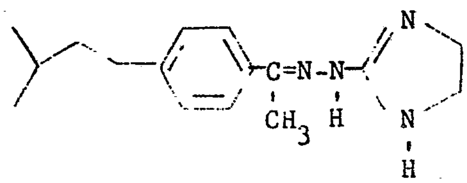 | base | 1051-29 |
| 24 | 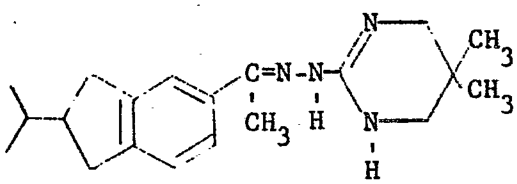 | base + bromhydrate | 1051-26 |
| 25 | 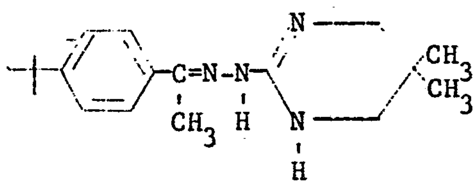 | base + bromhydrate | 1051-27 |
| 26 | 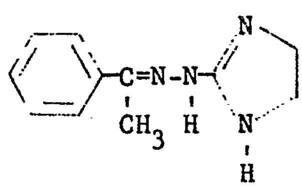 | bromhydrate | 1051-06 |
| 27 | 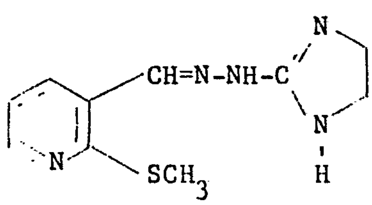 | bromhydrate | 1051-28 |
| 28 | 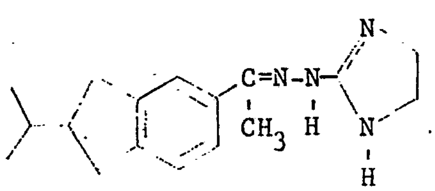 | phosphate | 1051-03 |

TABLEAU IX (suite 4)

| Exemple | | | |
|---|---|---|---|
| 29 | 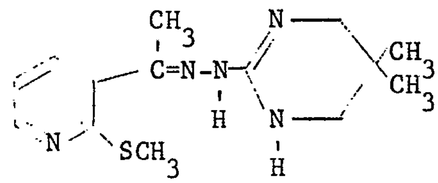 | bromhydrate | 1051-30 |
| 30 | 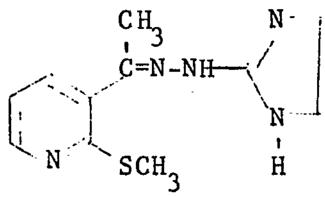 | base + bromhydrate | |
| I' | 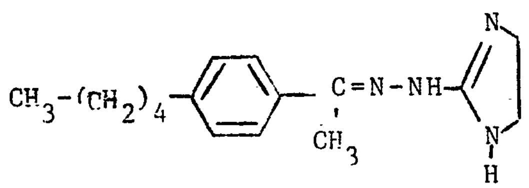 | base et bromhydrate | 1051-33 |
| II' | 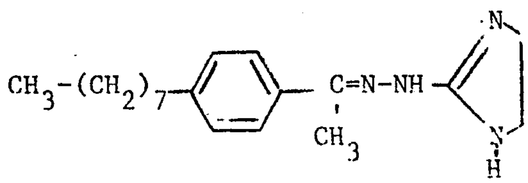 | base et bromhydrate | 1051-34 |
| III' | 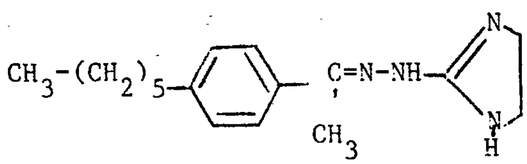 | base et bromhydrate | 1051-35 |
| IV' | 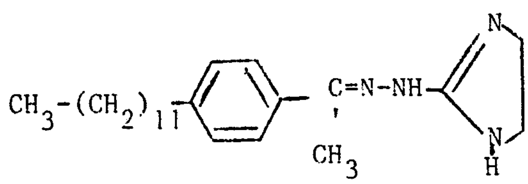 | base et bromhydrate | 1051-36 |
| V' | 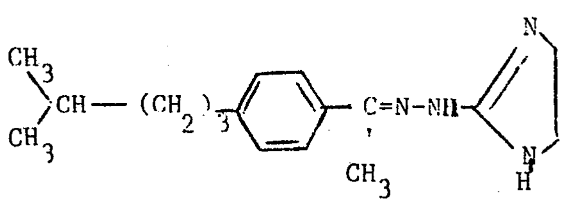 | base et bromhydrate | 1051-37 |
| VI' | 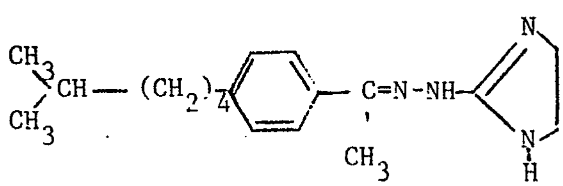 | base et bromhydrate | 1051-38 |

# R E V E N D I C A T I O N S

1. Nouveaux composés, caractérisés en ce qu'ils répondent à la formule générale (I)

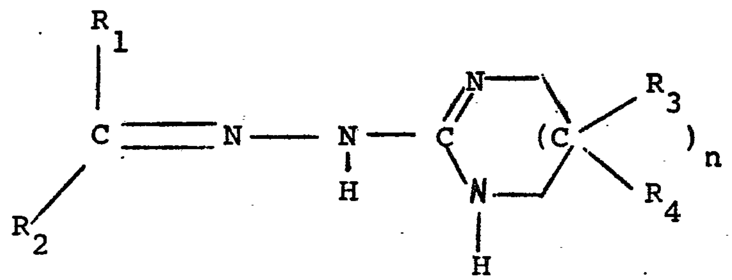

(I)

dans laquelle :

$R_1$ représente :

- un radical alkyle linéaire ou ramifié de 1 à 18 atomes de carbone ou un radical cyclo alkyle comprenant de 3 à 6 atomes de carbone, pouvant être substitué par un radical alkyle inférieur ;
- un groupement aryle de formule

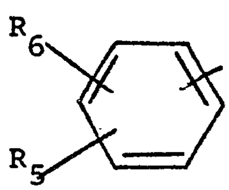

dans laquelle $R_5$ et $R_6$, qui sont identiques ou différents, représentent un atome d'hydrogène, un radical alkyle linéaire ou ramifié de 1 à 20 atome de carbone, un radical alkyloxy, un radical alkylthio, un radical alkényloxy, un radical cycloalkyle, un radical trifluorométhyle, un atome d'halogène, un radical aryle substitué ou non, un groupement aryloxy substitué ou non ;
- un groupement indanyl-5 substitué ou non en position 2 par un radical alkyle inférieur linéaire ou ramifié ;
- un radical naphtyl-1 ou naphtyl-2 substitué ou non par un groupement alkyle ou alkyloxy inférieur ;

- ou bien un hétérocycle azoté comme la pyridine ou la pyrimidine, substitué ou non par les radicaux $R_5$ et $R_6$ définis comme ci-dessus ;
- ou bien un hétérocycle soufré comme le cyclopentathiophène substitué ou non par un radical alkyle inférieur ;

$R_2$ représente :

- un atome d'hydrogène ou un radical alkyle inférieur ;

$R_1$ et $R_2$ pouvant former ensemble avec l'atome de carbone auquel ils sont reliés un cycloalkane, avantageusement le cyclopentane ou le cyclohexane, substitué ou non par un radical alkyle linéaire ou ramifié de 1 à 5 atomes de carbone ;

n vaut zéro ou un,

et dans le cas où n = 1 :

$R_3$ et $R_4$, identiques ou différents, représentent un atome d'hydrogène ou un radical alkyle inférieur;

et leur sels d'acides minéraux et organiques.

2. Nouveaux composés selon la revendication 1, caractérisés en ce qu'ils répondent à la formule (I) dans laquelle :

$R_1$ représente :

- un groupe aryle dans lequel $R_5$ représente un radical alkyle inférieur ou un radical cycloalkyle et $R_6$ représente un atome d'hydrogène ;
- un groupement indanyl-5 substitué en 2 par un radical alkyle inférieur ;
- un radical naphtyl-2 substitué en 6 par un groupement méthoxy ;

$R_2$ représente :

- un groupement méthyle;

n est égal à zéro.

3. Nouveaux composés selon la revendication 2 caractérisés en ce que $R_1$ représente un groupe aryle substitué en position para par un groupe isobutyle ou tert. butyle, et $R_2$ représente un radical alkyle inférieur.

4. Nouveaux composés selon la revendication 2 ou 3, caractérisés en ce que $R_1$ représente un radical indanyl-5.

5. Nouveau composé selon la revendication 1, caractérisé en ce qu'il consiste en la N-/¯(isopropyl-2' indanyl-5')-1 méthylène/ N'-(imidazolino-2) hydrazine.

6. Nouveau composé selon la revendication 1, caractérisé en ce qu'il consiste en la N-/¯(isopropyl-2' indanyl-5')-1 éthylidène/ N'-(imidazolino-2) hydrazine.

7. Nouveau composé selon la revendication 1, caractérisé en ce qu'il consiste en la N-/¯(isopropyl-2' indanyl-5')-1 propylidène/ N'-(imidazolino-2) hydrazine, et maléate correspondant.

8. Nouveau composé selon la revendication 1, caractérisé en ce qu'il consiste en l'acétate de N-/¯(indanyl-5')-1 éthylidène/ N'-(imidazolino-2) hydrazine.

9. Nouveau composé selon la revendication 1, caractérisé en ce qu'il consiste en l'acétate de N-/¯(indanyl-5')-1 propylidène/ N'-(imidazolino-2) hydrazine.

10. Nouveau composé selon la revendication 1, caractérisé en ce qu'il consiste en la N-/¯(terbutyl-4' phényl)-1 éthylidène/ N'-(imidazolino-2) hydrazine, et bromhydrate correspondant.

11. Nouveau composé selon la revendication 1, caractérisé en ce qu'il consiste en la N-/¯(isobutyl-4' phényl) -1 éthylidène/ N'-(imidazolino-2) hydrazine, et bromhydrate correspondant.

12. Nouveau composé selon la revendication 1, caractérisé en ce qu'il consiste en la N-/¯(cyclohexyl-4'

phényl)-1 éthylidène/ N'-(imidazolino-2) hydrazine, et bromhydrate correspondant.

13. Nouveau composé selon la revendication 1, caractérisé en ce qu'il consiste en la N-/(diphénylyl-4')-1 éthylidène/ N'-(imidazolino-2) hydrazine, et chlorhydrate correspondant.

14. Nouveau composé selon la revendication 1, caractérisé en ce qu'il consiste en la N-/(phénoxy-4' phényl)-1 éthylidène/ N'-(imidazolino-2) hydrazine, et chlorhydrate correspondant.

15. Nouveau composé selon la revendication 1, caractérisé en ce qu'il consiste en la N-/(naphtyl-1')-1 éthylidène/ N'-(imidazolino-2) hydrazine.

16. Nouveau composé selon la revendication 1, caractérisé en ce qu'il consiste en le Bromhydrate de N-/(méthoxy-6' naphtyl-2')-1 éthylidène/ N'-(imidazolino-2) hydrazine.

17. Nouveau composé selon la revendication 1, caractérisé en ce qu'il consiste en le Bromhydrate de N-(isopropylidène) N'-(imidazolino-2) hydrazine.

18. Nouveau composé selon la revendication 1, caractérisé en ce qu'il consiste en le Bromhydrate de N-/(isobutyl)-1 éthylidène/ N'-(imidazolino-2) hydrazine.

19. Nouveau composé selon la revendication 1, caractérisé en ce qu'il consiste en le Bromhydrate de N-/(n-pentyl)-1 éthylidène/ N'-(imidazolino-2) hydrazine.

20. Nouveau composé selon la revendication 1, caractérisé en ce qu'il consiste en la N-/(bromo-4' phenyl)-1 éthylidène/ N'-(imidazolino-2) hydrazine, et acétate correspondant.

21. Nouveau composé selon la revendication 1,

caractérisé en ce qu'il consiste en la N-[(chloro-4' phényl)-1 éthylidène] N' -(imidazolino-2) hydrazine, et acétate correspondant.

22. Nouveau composé selon la revendication 1, caractérisé en ce qu'il consiste en la N-[(tertiobutyl-4') cyclohexylidène] N'-(imidazolino-2) hydrazine, et bromhydrate correspondant.

23. Nouveau composé selon la revendication 1, caractérisé en ce qu'il consiste en la N-[(n-propyl-4' phényl)-1 éthylidène] N'-(imidazolino-2) hydrazine et bromhydrate correspondant.

24. Nouveau composé selon la revendication 1, caractérisé en ce qu'il consiste en la N-[(trifluoro méthyl-3' phényl)-1 éthylidène] N'-(imidazolino-2) hydrazine, et bromhydrate correspondant.

25. Nouveau composé selon la revendication 1, caractérisé en ce qu'il consiste en la N-[(éthyl-5' cyclopenta (b) thiényl-2')-1 éthylidène]N'-(imidazolino-2) hydrazine, et bromhydrate correspondant.

26. Nouveau composé selon la revendication 1, caractérisé en ce qu'il consiste en la N-[(phénoxy-3' phényl)-1 éthylidène] N'-(imidazolino-2) hydrazine et chlorhydrate correspondant.

27. Nouveau composé selon la revendication 1, caractérisé en ce qu'il consiste en la N-[(isopentyl-4' phényl)-1 éthylidène] N'-(imidazolino-2) hydrazine.

28. Nouveau composé selon la revendication 1, caractérisé en ce qu'il consiste en la N-[(isopropyl-2' indanyl-5')-1 éthylidène] N'-(diméthyl 5,5 tétrahydro- 3, 4,5,6 pyrimidino-2) hydrazine, et bromhydrate correspondant.

29. Nouveau composé selon la revendication 1, caractérisé en ce qu'il consiste en la N-/(tertio butyl-4' phényl)-1 éthylidène/ N'(diméthyl-5,5 tétrahydro-3, 4,5,6 pyrimidino-2) hydrazine et bromhydrate correspondant.

30. Nouveau composé selon la revendication 1, caractérisé en ce qu'il consiste en le Bromhydrate de N-/(phényl-1 éthylidène)/ N'-(imidazolino-2) hydrazine.

31. Nouveau composé selon la revendication 1, caractérisé en ce qu'il consiste en le Bromhydrate de N-/(méthylthio-2' pyridyl-3')-1 méthylène/ N'-(imidazolino-2) hydrazine.

32. Nouveau composé selon la revendication 1, caractérisé en ce qu'il consiste en le phosphate de N-/(isopropyl-2' indanyl-5')-1 éthylidène/ N'-(imidazolino-2) hydrazine.

33. Nouveau composé selon la revendication 1, caractérisé en ce qu'il consiste en le Bromhydrate de N-/(méthylthio-2' pyridyl-3')-1 éthylidène/ N'-(diméthyl-5,5 tétrahydro-3,4,5,6 pyrimidino-2) hydrazine.

34. Nouveau composé selon la revendication 1, caractérisé en ce qu'il consiste en la N-/(méthylthio-2' pyridyl-3')-1 éthylidène/ N'-(imidazolino-2) hydrazine et bromhydrate correspondant.

35. Nouveaux composés selon la revendication 1, caractérisés en ce qu'ils répondent à la formule générale (I') :

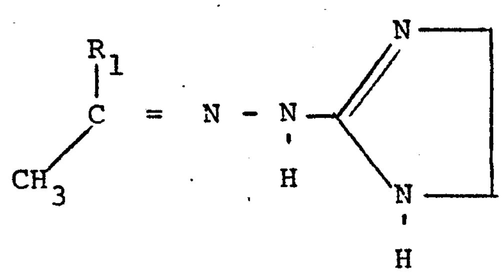

(I')

dans laquelle :

$R_1$ est un groupement aryle de formule :

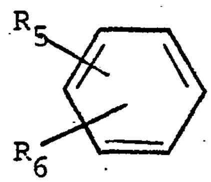

dans laquelle $R_5$ et $R_6$ qui sont identiques ou différents, représentent un atome d'hydrogène ou un radical alkyle linéaire ou ramifié de 5 à 20 atomes de carbone, ou un radical cycloalkyle,

et leurs sels d'acides minéraux ou organiques.

36. Nouveaux composés selon la revendication 35, caractérisés en ce que :

$R_1$ est : un groupe aryle dans lequel $R_5$ représente un radical alkyle linéaire ou ramifié de 5 à 20 atomes de carbone ou un radical cycloalkyle, et $R_6$ l'atome d'hydrogène.

37. Nouveaux composés selon la revendication 35 ou 36, caractérisés en ce que $R_5$ est en position para.

38. Nouveau composé selon la revendication 35 caractérisé en ce qu'il consiste en la N-[(n-pentyl-4' phényl)-1 éthylidène] N'-(imidazolino-2) hydrazine et son bromhydrate.

39. Nouveau composé selon la revendication 35, caractérisé en ce qu'il consiste en la N-[(n octyl-4' phényl)-1 éthylidène] N'-(imidazolino-2) hydrazine et son bromhydrate.

40. Nouveau composé selon la revendication 35, caractérisé en ce qu'il consiste en la N-[(n hexyl-4' phényl)-1 éthylidène]N'-(imidazolino-2) hydrazine et son bromhydrate.

41. Nouveau composé selon la revendication 35, caractérisé en ce qu'il consiste en la N-[(n dodécyl-4' phényl)-1 éthylidène] N'-(imidazolino-2) hydrazine et son bromhydrate.

42. Nouveau composé selon la revendication 35, caractérisé en ce qu'il consiste en la N-[(iso hexyl-4' phényl)-1 éthylidène] N'(imidazolino-2) hydrazine et son bromhydrate.

43. Nouveau composé selon la revendication 35, caractérisé en ce qu'il consiste en la N-[(iso heptyl-4' phényl)-1 éthylidène] N'(imidazolino-2) hydrazine et son bromhydrate.

44. Procédé de préparation des composés selon l'une quelconque des revendications 1 à 43, caractérisé en ce que l'on fait réagir un composé de formule (II) sur un composé de formule (III) dans un solvant organique, choisi principalement parmi les alcools et l'éthylène-glycol, à des températures comprises entre 60 et 130° C :

(II) 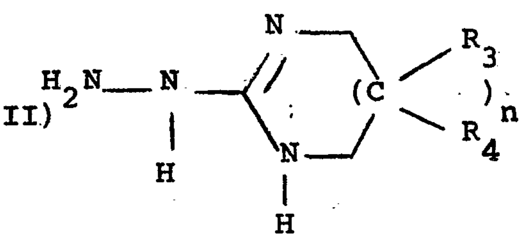

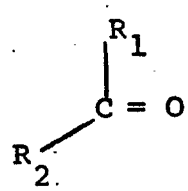 (III)

($R_1$, $R_2$, $R_3$, $R_4$ et n étant tels que définis dans la revendication 1 ou 35).

45. Utilisation des composés selon l'une quelconque des revendications 1 à 43 comme fongicides.

46. Utilisation selon la revendication 45 contre Fusarium roseum, Rhizoctonia solani, Phomopsis viticola, Alternaria tenuis, Uromyces phaseoli, Peronospora viticola, Botrytis fabae, Botrytis cinerea, Penicillium expansum, Aspergillus niger, Phytophthora infestans, Erysiphe graminis...

47. Utilisation selon la revendication 45 ou 46 à la dose de 50 à 2000 g/ha environ, notamment sous forme de poudres mouillables, poudres pour poudrage, suspensions aqueuses, solutions organiques.

48. Utilisation des composés selon l'une quelconque des revendications 1 à 43 comme bactéricides.

Office européen des brevets

0062587

Numéro de la demande

# RAPPORT DE RECHERCHE EUROPEENNE

EP 82 40 0619

| Catégorie | DOCUMENTS CONSIDERES COMME PERTINENTS<br>Citation du document avec indication, en cas de besoin, des parties pertinentes | Revendication concernée | CLASSEMENT DE LA DEMANDE (Int. Cl. 3) |
|---|---|---|---|
| X | FR - A - 2 337 550 (HEXACHIMIE)<br>* pages 1,2,4,5,11 *<br>-- | 23,44,<br>1-3,<br>10,11 | C 07 D 233/52<br>239/18<br>401/12<br>403/12<br>409/12<br>A 01 N 43/50<br>43/54 |
| X | FR - M - 8343 (AMERICAN HOME)<br>* pages 2-4, 10-13, 17 *<br>-- | 1,44,<br>48 | |
| X | AU - B - 57 138/73 (SANDOZ)<br>* pages 2-4 *<br>-- | 1,44 | DOMAINES TECHNIQUES RECHERCHES (Int. Cl. 3) |
| X | CHEMICAL ABSTRACTS, volume 82, no. 25, 23 juin 1975, page 555, abrégé 171024h<br>COLUMBUS OHIO (US)<br>& JP - B - 74 125 374 (TAKEDA) (30-11-1974)<br>* l'abrégé *<br>----------- | 1,44 | C 07 D 233/00<br>239/00<br>401/00<br>403/00<br>409/00 |

Le présent rapport de recherche a été établi pour toutes les revendications

| Lieu de la recherche | Date d'achèvement de la recherche | Examinateur |
|---|---|---|
| La Haye | 11-06-1982 | DE BUYSER |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503.03.82